(19) **Europäisches Patentamt**

**European Patent Office**

**Office européen des brevets**

(11) **EP 1 339 360 B1**

(12) **EUROPEAN PATENT SPECIFICATION**

(45) Date of publication and mention
of the grant of the patent:
**05.07.2006 Bulletin 2006/27**

(51) Int Cl.:
**A61F 2/16** (2006.01)     **G02B 5/18** (2006.01)

(21) Application number: **00982212.3**

(22) Date of filing: **27.11.2000**

(86) International application number:
**PCT/US2000/032148**

(87) International publication number:
**WO 2002/041806 (30.05.2002 Gazette 2002/22)**

(54) **FOLDABLE THIN INTRAOCULAR MEMBRANE**

FALTBARE, DÜNNE INTRAOKULARE MEMBRAN

MEMBRANE INTRA-OCULAIRE MINCE, PLIABLE

(84) Designated Contracting States:
**AT BE CH CY DE DK ES FI FR GB GR IE IT LI LU
MC NL PT SE TR**

(43) Date of publication of application:
**03.09.2003 Bulletin 2003/36**

(73) Proprietor: **Nordan, Lee T.
La Jolla, CA 92037 (US)**

(72) Inventor: **Nordan, Lee T.
La Jolla, CA 92037 (US)**

(74) Representative: **Viering, Jentschura & Partner
Steinsdorfstrasse 6
80538 München (DE)**

(56) References cited:
**EP-A- 0 605 841          WO-A-00/66039
WO-A-00/67678          CA-A- 2 093 097
NN-A-                          US-A- 4 753 998
US-A- 4 828 558          US-A- 6 015 435
US-A- 6 096 077          US-A- 6 152 958**

**Description**

**Field of the Invention**

The invention relates to a single piece corrective device according to the preamble of claim 1.

Accordingly, the present invention generally relates to intraocular implants, and more specifically to implants intended to be inserted in the anterior chamber of the eye in order to correct optical deficiencies without removal of the crystalline lens.

**Background of the Invention**

A device of the above-mentioned type is known, e.g., from WO 00/660390.

Intraocular lenses (IOLs) are routinely used nowadays for restoring vision after removal of the cataracted lens. Whether the IOL is installed in the posterior chamber of the eye in lieu of the removed cataracted lens, or in the anterior chamber, it must be small enough to pass through a minimal corneal incision. The reduction in the overall dimension of the IOL is limited, however, by the necessity of avoiding glare by providing a substitute optic that is large enough to cover the pupil when it is fully dilated for proper night time vision. One approach to reducing glare while at the same time reducing the size of the incision in the cornea is to construct the IOL from several pieces which are joined together after the individual pieces are inserted through the corneal incision as disclosed in U.S. Patent No. 5,769,889 Kelman. The complexity of this type of IOLs, the difficulty of their post insertion assembly coupled with the required thickness and rigidity of the optic element, still force the ophthalmic surgeon into tolerable compromises between reduced size and peripheral glare coupled with impaired night vision.

Conventional lenticular elements, whatever their size, are still subject to various spherical and thickness aberrations which are not easily correctable during the manufacture of the IOL.

A deformable, artificial intraocular contact lens for implantation into the human eye to correct normal-vision problems is disclosed in US 6,096,077. Therein, the lens may be positioned posteriorly from the iris, resting against the anterior surface of the posterior capsule's natural lens, or alternatively, the lens may be positioned in the anterior chamber of the eye. The implanted lens works in conjunction with the cornea and natural lens to provide proper vision, as a substitute for regular contact lens, spectacles, and radial keratotomy.

The invention results from a search for a simple, preferably one-piece IOL with an optic having a diameter sufficient to cover the size of a dilated pupil, but yet insertable to a relatively small corneal incision.

Summary of the Invention

The principal and secondary objects of this invention are to provide the ophthalmic surgeon with a simple, one-piece IOL which avoids the major drawbacks of the devices of the prior art, particularly reduced coma, glare, impaired night vision, and blurring due to spherical and thickness aberrations, and which can be collapsed to a relatively small size for insertion through a corneal incision of about 2.75 millimeters.

This is achieved by a single piece corrective device having the features of claim 1.

Preferred embodiments of this invention are described in the dependent claims.

These and other valuable objects are achieved by forming a thin lens inherently immune to spherical and thickness aberrations on a resiliently flexible membrane that can be rolled or folded to pass through a small corneal incision. The thin lens optic typically uses a small central lenticular zone surrounded by a plurality of optic rings concentric with the central zone and extends up to a total diameter of approximately 6 millimeters. In a first embodiment of the invention, the lenticular zone forms a refractive Fresnel-type lens. An alternate embodiment of the invention features a diffractive phase Fresnel-type lens formed of concentric zones having profiles that provide a phase jump delay at each zone boundary which is a multiple of waves at the design wavelength in order to focus a plurality of different wavelengths to a single point. The membrane and its incorporated thin lens optic is arcuately shaped for installation in the anterior chamber where it vaults the iris and is stabilized by sets of obliques flaps that nest into the corner of the chamber. Contrary to the compressed haptics commonly used to secure prior art, IOLs, the aforesaid flaps do not exert any pressure upon the wall of the eye. The vaulted shape of the device combined with its thinness keep it away from the endothelium. Its neutral buoyancy prevents any pressure on the iris eliminating risks of closure, cataract or iris pigment dispersion. The large footprint of the flaps prevent synechiae and their encapsulation by the iris. The natural buoyancy of the device is improved by a plurality of fenestrations. The thin lens can be configured in a variety of successive dioptic powers, over a range from -15 to +15 diopters, in order to correct practically all types of refractive errors.

## Brief Description of the Drawings

**Figure 1** is a front elevational view of the preferred embodiment of the invention;
**Figure 2** is a bottom plan view thereof;
**Figure 3** is a side elevational view thereof;
**Figure 4** is a diagrammatical illustration of the device implantation in the anterior chamber of the eye;
**Figure 5** is a cross-sectional view of a thin lens optic region;
**Figure 6** is a front elevational view of a first alternate embodiment of the invention;
**Figure 7** is a bottom plan view thereof;
**Figure 8** is a side elevational view thereof;
**Figure 9** is a front elevational view of a second alternate embodiment of the invention;
**Figure 10** is a bottom plan view thereof;
**Figure 11** is a side elevational view thereof;
**Figure 12** is a front elevational view of third alternate embodiment of the invention;
**Figure 13** is a bottom plan view thereof;
**Figure 14** is a side elevational view thereof;
**Figure 15** is a diagram of a prior art MOD lens;
**Figure 16** is a diffraction plot thereof;
**Figure 17** is a diagram of an optical system of a MOD lens; and
**Figure 18** is a diagram of the diffractive pattern of the alternate optic embodiment of the invention.

## Description of the Preferred Embodiment of the Invention

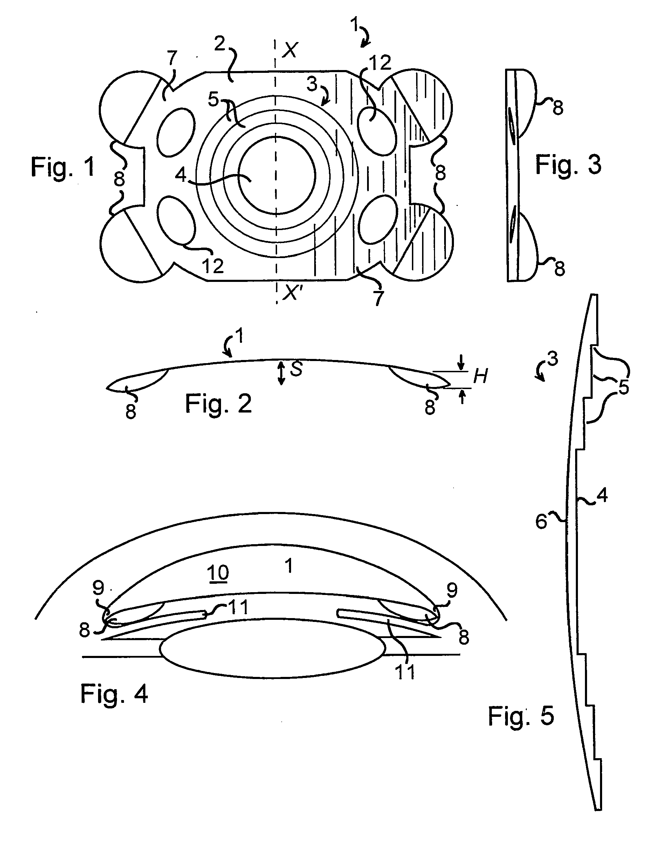

Referring now to Figures 1-14, there is shown a refractively corrective device 1 shaped and dimensioned for installation in the anterior chamber of a phakic or pseudophakic eye. The device consists essentially of a membrane preferably made of flexible polymethylmethacrylate (PMMA). Other resilient materials such as silicone or hydrogel could also be used. The overall dimensions are approximately 12 millimeters in length, 8 millimeters in width and an overall thickness of approximately 10 to 100 microns. The membrane can be bent and even rolled or folded for insertion into the interior chamber through a small incision of no more than 2.75 millimeters in length. The membrane has enough resiliency to return to its prerolled or prefolded shape. The optic region 2 in the center of the membrane has an overall diameter of approximately 6 millimeters. The optic region is essentially constituted by what is called a "thin lens" in the fields of optics and ophthalmology. A thin lens is a refractive device 3 of the type illustrated in Figure 4 that combines a lenticular central zone 4 with a plurality of refractive rings 5 surrounding and concentric with the lenticular zone 4. In some applications, the entire optical element may be constituted by concentric rings. Those concentric rings resemble the rings of a Fresnel lens, except that instead of defining a prism, each ring has a front surface 6 espousing a continuous curve in common with the front surface of the central lenticular zone 4. It should be noted that this curved front surface may be convex, or concave depending upon the type of correction required and is seldom spherical, but may exhibit various degrees of asphericity as required to provide both a single diopter correction or multi-diopter correction as previously taught in my U.S. Patent No. 5,236,452 issued November 21, 1991. The optic is typically made by machining the PMMA membrane with a laser milling machine. The two lateral portions 7 of the membrane astride the median optic region 2 are shaped to define at least two flaps 8 which are permanently bent obliquely backward in relation to the median region in order to nest intimately into the corner 9 of the anterior chamber as illustrated in Figure 3. Accordingly, the median portion and the lateral portion with their bent flaps 8 form a vault having a total sagital length S of approximately 1 millimeter whereby the membrane 1 spans the anterior chamber 10 in a direction substantially parallel to the iris 11. It should be noted that the median portion and the lateral portions exclusive of the flaps may be slightly arcuate about the transversal length central axis XX' to a total sagital length of approximately 0.5 millimeter. In which case, the height of the flaps H would be reduced to approximately 0.5 millimeter in order to obtain a total vault height or sagital length S of approximately 1 millimeter. The curvature of the membrane and the bent of flaps are permanently imparted during the fabrication of the device.

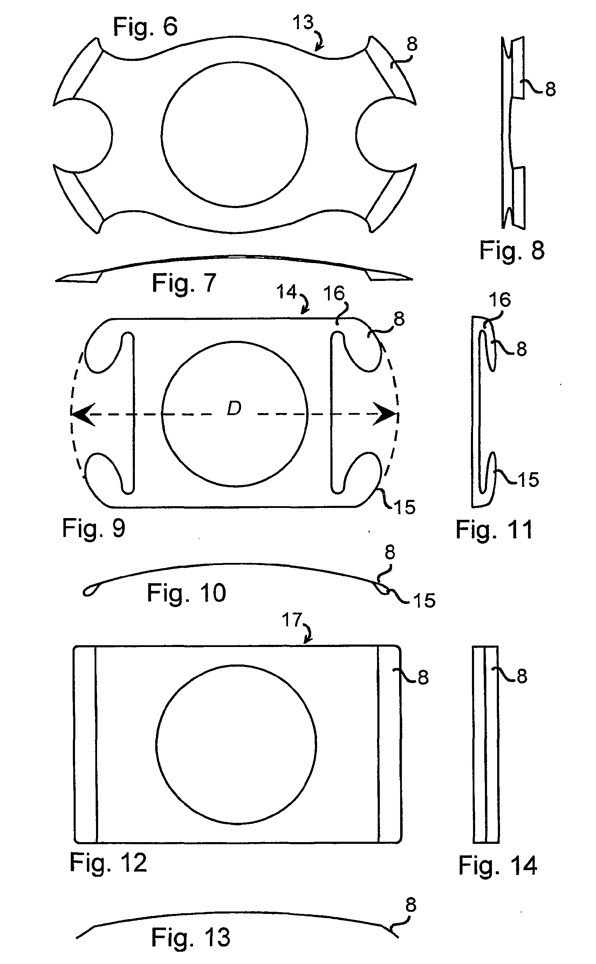

Alternate embodiments of the device are illustrated in Figures 6-14. In each of these alternate embodiments, the membrane returns to its arcuate shape and backward projecting flaps 9. Each device fits within a circle having a diameter D of approximately 13 millimeters. Due to the flexibility of the membrane, this size can accommodate practically all eye sizes.

In the embodiment 13 of Figures 6-8, the surface of the membrane is reduced for maximum buoyancy.

In the embodiment 14 of Figures 9-11, the flaps have an increased thickness along their edges 15 to create a larger footprint against the wall of the eye. The collar regions 16 between each flap and the main body of the membrane is thinned down to about 20 microns in order to provide flexibility with a modicum amount of resiliency.

The embodiment 17 of Figures 12-14 is of a simplified shape that can provide an even larger optic zone.

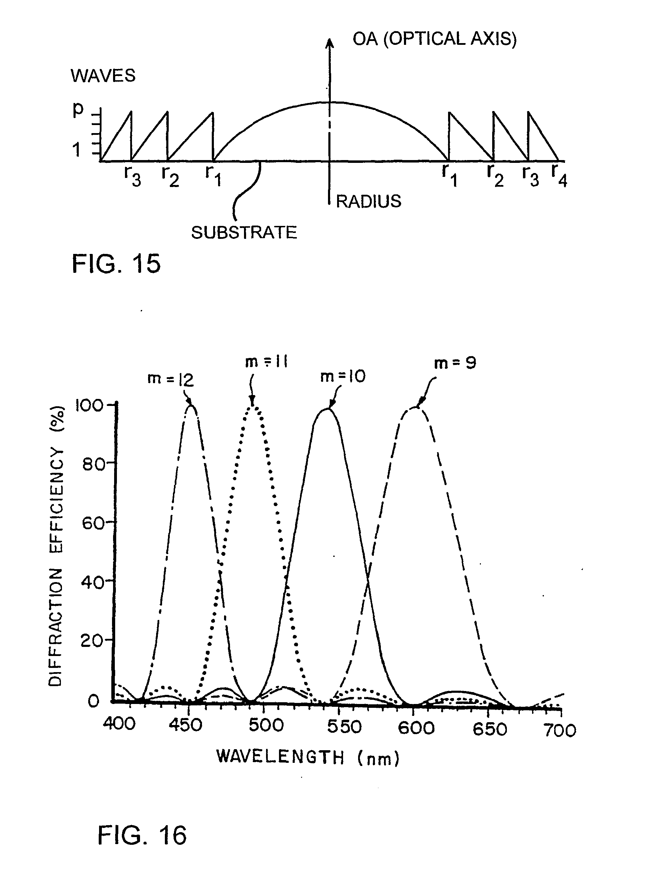

Referring now to Figures 15-18, in an alternate embodiment of the thin lens implant of the invention, the optic is constituted by a multi-order diffractive (MOD) lens having a surface geometry of the type disclosed in U.S. Patent No. 5,589,982 Faklis et al.

As disclosed in said patent, a MOD lens is capable of focusing a plurality of different wavelengths of light to a single focus. A diffractive structure is used having a plurality of annular zones which define zone boundaries which diffract light of each of the wavelengths in a different diffractive order to the focus thereby providing a plural or multiple order diffractive singlet.

The imaging properties of a plural or multi-order diffractive (MOD) lens enable the use of the lens in conjunction with light that has either a broad spectral range or a spectrum consisting of multiple spectral bands. The MOD lens differs from standard diffractive lenses in that the phase delay or jump at the zone boundaries is a multiple of waves at the design wavelength (a multiple of $2\pi$, i.e., $\phi(r_j)=2\pi p$, where p is an integer greater or equal to 2, and the zone radii are obtained by solving the equation $\phi(r_j)=2\pi pj$, where $\phi(r)$ represents the phase function for the wavefront emerging from the lens). The number of $2\pi$ phase jumps, p, represents a degree of freedom allowing an optical designer to use distinct diffraction orders to focus two or more spectral components upon the same spatial location.

Referring to Figure 15, the blaze profile is on one side of a substrate of optically transmissive material. The number of waves for each zone boundary is indicated as p and the phase jump of phases at each zone boundary, which are at radii r1, r2, r3 andr4 is constant. The center of the lens is along the optical axis and is perpendicular to the plane of the planar substrate on which the profile is formed. The profile of the lens may also be a phase reversal (or Wood) profile, or a multilevel approximation to the blaze profile.

The profile may be defined between substrates, rather than on a planar surface of a substrate, as shown, where the substrates on opposite sides of the profile have different indicies of refraction. The profile may be defined on a curved substrate.

The zone spacing or width of the zones between the zone boundaries $r_1$-$r_2$, $r_2$-$r_3$, $r_3$-$r_4$ are full period Fresnel zones. These zones are defined such that the optical path difference across the jth zone is equal to "$P\lambda_0$" where $\lambda_0$ is the design wavelength, $F_0$ is the focal length, when the illumination wavelength $\lambda = \lambda_0$ and P is an integer that represents the maximum phase modulation as a multiple of $2\pi$. In the paraxial region, the locations of the zones in the plane of the lens are given by

$$r^2_j 2jp\lambda_o F_o \qquad (1)$$

The optical phase introduced by the diffractive element is given by

$$\phi(r) = 2\pi\alpha p \left[ j - \frac{r^2}{2p\lambda_0 F_0} \right] , r_j \le r < r_{j-1} \qquad (2)$$

where $\alpha$ is defined as the fraction of $2\pi$ phase delay that is introduced for illumination wavelengths other than the design wavelength and is given by

$$\alpha = \frac{\lambda_o}{\lambda} \left[ \frac{n(\lambda) - 1}{n(\lambda_{o)} - 1} \right] \qquad (3)$$

where n is the index of refraction of the material in the grating region. The maximum height of the surface relief is given by

$$h_{max}(r) = \frac{p\lambda_0}{n(\lambda_0) - 1} \qquad (4)$$

The amplitude transmission function of the diffractive lens can be expanded as a Fourier series to give

$$t(r) = \sum_{m=-\infty}^{\infty} e^{-i\pi(\alpha p - m)} \sin c(\alpha p - m) e^{-\frac{i\pi r^2 m}{p\lambda_o F_o}} \quad (5)$$

where sinc(x)=sin(πx)/(πx) and m denotes the m-th diffraction order. The transmission function in Eq. (5) represents a diffractive lens within the paraxial approximation.

$$F(\lambda) = \frac{p\lambda_o F_o}{m\lambda} \quad (6)$$

The focal length in Eq. (6) is proprotional to p and inversely proportional to the illumination wavelength and the diffraction order, m. When the quantity in Eq. (6), $p\lambda_o/m\lambda$, is set equal to unity, several wavelengths can come to a common focus. The p letter represents a construction parameter and is usually constant across the lens radius, and the wavelengths that are focused to a common point are chosen from a set of diffraction orders (i.e., multi-order diffractive lenses). While a diffractive lens with a maximum phase modulation of 2π can allow a mutual focus for simple harmonics of the design wavelength, the parameter p offers a mechanism to control specific wavelengths in a given band or bands that will come to a fixed focus. This property allows the design of achromats and apochromats using a single diffractive surface.

The scalar diffraction efficiency, $\eta_m$ of the m-th diffracted order is given by the squared modulus of its Fourier coefficient in Eq. (5), i.e.

$$\eta_m = sinc^2(\alpha p - m) \quad (7)$$

The diffraction efficiency given by Eq. (7) is unity when the argument of the sinc function is equal to zero. Notice that this condition can allow for high diffraction efficiency for several wavelengths. For example, consider the case of a multi-order diffractive lens operating in the visible region with p=10. Figure 16 illustrates the wavelength dependence of the diffraction efficiency for a range of diffracted orders neglecting material dispersion. The peaks in diffraction efficiency occur at precisely those wavelengths that come to a common focus (see Eq. (6), i.e.,

$$\lambda_i = \frac{p\lambda_o}{m} \quad (8)$$

The wavelength bandwidth of the diffraction efficiency around a given diffracted order narrows with increasing values of p (see Eq. (7) and Figure 16).

Using Eq. (8), is is possible to choose the parameters p and m to design a diffractive singlet that has high diffraction efficiency for specific bands in a given spectrum. The center wavelength of each of these bands comes to a focus distance $F_o$ behind the lens.

It has been shown above that a multi-order diffractive (MOD) lens offers the potential for several wavelength components of the incident spectrum to come to a common focus. As a result, the polychromatic performance of the MOD signlet will differ substantially from the typical diffractive lens operating in a single diffraction order. Using the diffractive lens transmission function given in Eq. (5), the optical transfer function (OTF) for the paraxial diffractive singlet as a function of wavelength may be calculated. Only the effects of the wavelength-dependent defocus and not any higher-order aberrations need to be included, since these aberrations can be accounted for by incluidng appropriate phase terms in the diffraction integrals.

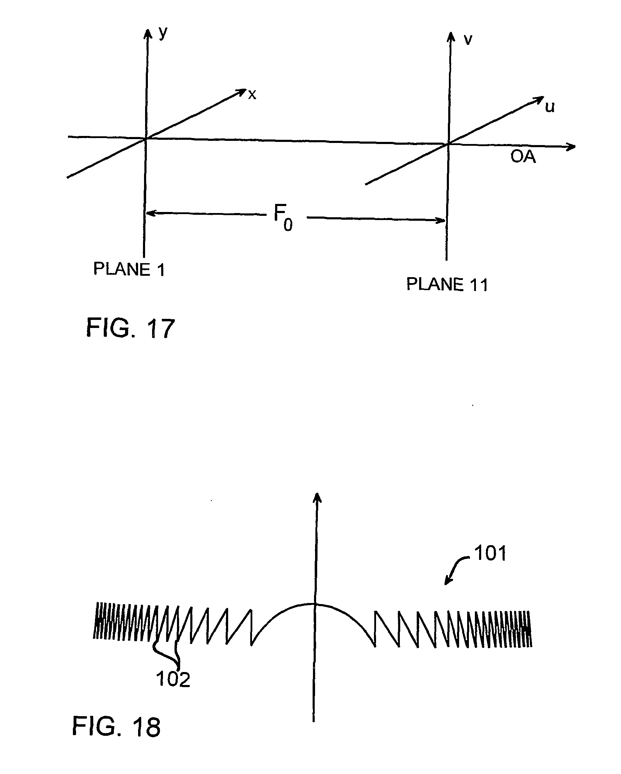

Figure 17 illustrates the geometry to compute the field distribution in the rear focal plane, which is given by $U_{II}(u,v;\lambda) =$

$$\frac{-i}{\lambda F_0}\iint_{-\infty}^{\infty}U_I(x,y;\lambda)e^{\frac{i\pi}{\lambda F_0}[(u-x)^2(v-y)^2]}dxdy \tag{9}$$

where (u,v) represent the coordinates in plane II located a distance $F_O$ away and P(x,y) is the complex pupil function. Substituting Eq. (5) into Eq. (9) and assuming a unit-amplitude plane wave as the input gives

$$U_{II}(u,v;\lambda)=\frac{-ie^{\frac{i\pi}{\lambda F_0}(u^2+v^2)}}{\lambda F_0}\sum_{m=-\infty}^{\infty}e^{-i\pi(\alpha p-m)}\sin c(\alpha p-m)\cdot \int\int_{-\infty}^{\infty}P(x,y;\lambda)e^{\frac{-i\pi}{\lambda}(x^2+y^2)}e^{\frac{-i2\pi}{\lambda F_0}(ux+vy)}dxdy \tag{10}$$

where

$$\epsilon=\frac{1}{F_0}\left[\frac{m\lambda}{p\lambda_0}-1\right] \tag{11}$$

From Eq. (10), it can be seen that the magnitude of the quantity e, is related directly to the amount of defocus in plane II. If other aberrations were present, one could construct a generalized pupil function in Eq. (10) that includes the phase errors introduced by the aberrations. The OTF is given by the autocorrelation of the generalized pupil function, suitably normalized,

$$OTF(f_x,f_y)=\frac{\iint_{-\infty}^{\infty}H\left(\xi+\frac{f_x}{2},\eta+\frac{f_y}{2}\right)H^*\left(\xi-\frac{f_x}{2},\eta-\frac{f_y}{2}\right)d\xi d\eta}{\iint_{-\infty}^{\infty}|H(\xi,\eta)|^2 d\xi d\eta} \tag{12}$$

where H represents the coherent transfer function of the system. Assuming a rectangular pupil for simplicity, and using Eq. (10), the OTF as a function of the spectral frequencies $f_x$, $f_y$, is given by

$$OTF(f_x,f_y)=\Lambda\left(\frac{f_x}{2f_0}\right)\Lambda\left(\frac{f_y}{2f_0}\right)\sum_{-\infty}^{\infty}\sin c^2(\alpha p-m)\ \sin c\left[\frac{\epsilon l^2}{\lambda}\left(\frac{f_x}{2f_0}\right)\left(1-\frac{|f_x|}{2f_0}\right)\right] \tag{13}$$

$$\sin c\left[\frac{\epsilon l^2}{\lambda}\left(\frac{f_y}{2f_0}\right)\left(1-\frac{|f_y|}{2f_0}\right)\right]$$

where I is the diameter of the pupil aperture and

$$f_0 = \frac{1}{2\lambda F_0}, \qquad (14)$$

represents the cutoff frequency. In the derivation of Eq. (13) using Eq. (12), the cross terms in the double summation vanish due to an orthogonality condition for the diffracted orders. With reference to Eq. (13), note that the cases when p=m=a=1 and when p=m and a-1, the OTf reduces to

$$OTF(f_x, f_y) = \Lambda\left(\frac{f_x}{2f_0}\right)\Lambda\left(\frac{f_y}{2f_0}\right) \qquad (15)$$

which represents the diffraction-limited OTF associated with a rectangular aperture.

Equation (13) illustrates the imaging characteristics of the MOD lens. It contains three main terms: a) triangle functions that represent the diffraction-limited portion of the OTF; b) the $sinc^2$ function, which is the diffraction efficiency in a given order, acts as a weighting function; and c) the sinc terms that represent the degradation in the OTF due to defocus. It is important to note that the weighting function peaks to a value of unity whenever the argument is equal to zero. For the case of p=1, the typical diffractive lens in the visible wavelength range $\lambda_0/\lambda$ =m; only the design wavelength will have high diffraction efficiency. Furthermore, the sinc terms in the OTF illustrate that only the design wavelength will come to a sharp focus in the image plane (i.e. E is zero) and all other wavelength components in the band will be severely defocused (i.e., E is non-zero). For this case when p>2, the MOD lens can allow several wavelengths to have high diffraction efficiency (i.e.,$\lambda_0/\lambda$ =m) and a common focus (i.e., e can be zero for several discrete wavelengths). Equation (13) describes how a MOD lens can provide superior performance in broadband or multi-spectral illumination.

The previous imaging examples describe the effects of a wavelength-dependent defocus, but do not describe the effects of off-axis aberrations. In general, the MOD lens will have different wavelength-dependent aberration properties than the conventional diffractive singlet (i.e., p=1). However, for the important case of wide-field diffractive lenses, one finds that the third-order aberration properties of the MOD lens are identical to those found for the conventional wide-field diffractive singlet. For example, a MOD landscape lens (indefinite-conjugate imaging) will have no coma, astigmatism, and Petzval curvature for each wavelength that satisfies the equation $\lambda_{op}/\lambda$ =m; hence, a MOD lens is able to provide high quality imaging in broadband or multi-spectral illumination over wide fields of view.

As illustrated in the diagram of Figure 18, representing the diffractive pattern **101** of a polychromatic MOD lens that forms the optic of the alternate embodiment of the invention, the zone boundaries 102 diffract light of each wavelength in a different diffractive order to a single focus point. The phase delay or jump at the zone boundaries is a multiple of waves at the desired wavelength so that

$$\phi(r_j) = 2\pi p,$$

where p is an integer greater or equal to 2,
and the location of radii r of a zone j, is determined by the equation.

$$\phi(r_j) = 2\pi p_j$$

where $\phi(r)$ represent the phase function for the wavefront emerging from the lens. The number p of $2\pi$ phase jumps corresponds to the number of diffraction orders that can be used to bring two or more spectral components to focus at the same special location.

Example I:

A thin, foldable, MOD diffractive, polychromatic, intra-ocular implant according to the invention has been implemented based upon the following parameters and was shown to exhibit the following characteristics.
Material: PMMA

Thickness: 121 microns
Diameter of corrective portion: 0.65 centimeter
Power: -6 diopters
Number of concentric zones: 50
Radial location and width of each zone:

| Zone Number | Phase | Radial Location (millimeters) | Width (microns) |
|---|---|---|---|
| | 0 | 0.00000 | 0.000 |
| 1 | 1 | 0.42564 | 525.636 |
| 2 | 2 | 0.60194 | 176.305 |
| 3 | 3 | 0.73722 | 135.283 |
| 4 | 4 | 0.85127 | 114.049 |
| 5 | 5 | 0.95176 | 100.479 |
| 6 | 6 | 1.04259 | 90.840 |
| 7 | 7 | 1.12613 | 83.537 |
| 8 | 8 | 1.20388 | 77.753 |
| 9 | 9 | 1.27691 | 73.028 |
| 10 | 10 | 1.34598 | 69.071 |
| 11 | 11 | 1.41168 | 65.696 |
| 12 | 12 | 1.47445 | 62.772 |
| 13 | 13 | 1.53466 | 60.206 |
| 14 | 14 | 1.59259 | 57.931 |
| 15 | 15 | 1.64848 | 55.898 |
| 16 | 16 | 1.70255 | 54.063 |
| 17 | 17 | 1.75495 | 52.398 |
| 18 | 18 | 1.80582 | 50.878 |
| 19 | 19 | 1.85531 | 49.484 |
| 20 | 20 | 1.90351 | 48.198 |
| 21 | 21 | 1.95051 | 47.007 |
| 22 | 22 | 1.99641 | 45.901 |
| 23 | 23 | 2.04128 | 44.869 |
| 24 | 24 | 2.08519 | 43.903 |
| 25 | 25 | 2.12818 | 42.998 |
| 26 | 26 | 2.17033 | 42.147 |
| 27 | 27 | 2.21167 | 41.343 |
| 28 | 28 | 2.25226 | 40.585 |
| 29 | 29 | 2.29212 | 39.866 |
| 30 | 30 | 2.33131 | 39.184 |
| 31 | 31 | 2.36985 | 38.537 |
| 32 | 32 | 2.40777 | 37.920 |
| 33 | 33 | 2.44510 | 37.332 |
| 34 | 34 | 2.48187 | 36.770 |
| 35 | 35 | 2.51810 | 36.234 |
| 36 | 36 | 2.55382 | 35.719 |
| 37 | 37 | 2.58905 | 35.227 |
| 38 | 38 | 2.62380 | 34.754 |
| 39 | 39 | 2.65810 | 34.299 |
| 40 | 40 | 2.69196 | 33.863 |
| 41 | 41 | 2.72541 | 33.442 |
| 42 | 42 | 2.75844 | 33.036 |
| 43 | 43 | 2.79109 | 32.646 |
| 44 | 44 | 2.82336 | 32.267 |
| 45 | 45 | 2.85526 | 31.904 |

Table continued

| Zone Number | Phase | Radial Location (millimeters) | Width (microns) |
|---|---|---|---|
| 46 | 46 | 2.88681 | 31.551 |
| 47 | 47 | 2.91802 | 31.209 |
| 48 | 48 | 2.94890 | 30.880 |
| 49 | 49 | 2.97946 | 30.559 |
| 50 | 50 | 3.00791 | 30.249 |

While the preferred embodiments of the invention have been described, modifications can be made and other embodiments may be devised without departing from the scope of the appended claims.

## Claims

1.  A single piece corrective device (1) for installation in the anterior chamber (10) of a phakic or pseudophakic eye which comprises: a single resiliently bendable membrane having a substantially constant thickness of approximately 10 to 125 microns and shaped and dimensioned to span the anterior chamber (10) substantially in parallel to the iris (11); said membrane comprising a corrective median portion (2), and at least two lateral portions (7) astride said corrective median portion (2);
    **characterized in that**
    said corrective median portion (2) includes a multi-order, diffractive lens, said lens comprising a discontinuous optic zone (3) having a plurality of concentric optic rings (5); and
    each of said lateral portions (7) comprises at least two oblique anchoring flaps (8) each shaped and dimensioned to intimately nest into a corner (9) of the anterior chamber (10), wherein the flaps (8) are permanently bent obliquely backwards so that a vault having a sagittal length of approximately 1 millimeter is formed.

2.  The device (1) of Claim 1, wherein said membrane is made of flexible silicone.

3.  The device (1) of Claim 1, wherein said lens has correction powers in a range of approximately minus 15 diopters to plus 15 diopters.

4.  The device (1) of Claim 1, wherein each of said plurality of concentric optic rings (5) has a ring boundary with an adjacent ring and said rings (5) are shaped and dimensioned to provide a phase jump at each ring boundary for at least one spectral component of a light beam incident upon said lens.

5.  The device (1) of Claim 4, wherein a plurality of said spectral components have a given wavelength and said rings (5) are shaped and dimensioned to provide a phase jump equal to 2np wherein p is an integer greater or equal to 1, said p and the widths of the rings (5) are selected to direct said spectral components to a single focus point.

6.  The device (1) of Claim 5, wherein said rings (5) are radially spaced at radii, $r_j$, obtained by solving the equation $\phi(r_j) = 2np$ where $\phi(r)$ represents the phase function of a wavefront emerging from said optic rings (5).

7.  The device (1) of Claim 1, having sufficient flexibility to adjustably change said radius to match the span of said anterior chamber (10).

## Patentansprüche

1.  Einstückige Korrekturvorrichtung (1) zum Installieren in der anterioren Kammer (10) eines phaken oder pseudophaken Auges, welche aufweist: eine einzelne, elastisch biegbare Membran, die eine im Wesentlichen konstante Dicke von etwa 10 bis 125 Mikrometern aufweist und die so geformt und so dimensioniert ist, dass sie im Wesentlichen parallel zu der Iris (11) die anteriore Kammer (10) überspannt, wobei die Membran einen Korrektur-Mittelabschnitt (2) und wenigstens zwei Seitenabschnitte (7) beidseits des Korrektur-Mittelabschnitts (2) aufweist, **dadurch gekennzeichnet,**
    **dass** der Korrektur-Mittelabschnitt (2) eine Mehrordnungs-Diffraktions-Linse aufweist, wobei die Linse eine diskontinuierliche, optische Zone (3) mit einer Mehrzahl von konzentrischen, optischen Ringen (5) aufweist, und

**dass** jeder der Seitenabschnitte (7) wenigstens zwei schräge Befestigungslaschen (8) aufweist, die jeweils so geformt und so dimensioniert sind, dass sie genau in eine Ecke (9) der anterioren Kammer (10) passen, wobei die Laschen (8) dauerhaft schräg nach hinten gebogen sind, so dass eine Wölbung mit einer Sagittallänge von etwa 1 Millimeter ausgebildet ist.

**2.** Vorrichtung (1) gemäß Anspruch 1, wobei die Membran von flexiblem Silikon gebildet ist.

**3.** Vorrichtung (1) gemäß Anspruch 1, wobei die Linse Korrekturstärken in einem Bereich von etwa minus 15 Dioptrien bis plus 15 Dioptrien aufweist.

**4.** Vorrichtung (1) gemäß Anspruch 1, wobei jeder der Mehrzahl von konzentrischen, optischen Ringen (5) eine Ringgrenze zu einem benachbarten Ring aufweist, und wobei die Ringe (5) so geformt und so dimensioniert sind, dass sie an jeder Ringgrenze einen Phasensprung für wenigstens eine Spektralkomponente eines auf die Linse einfallenden Lichtstrahls bereitstellen.

**5.** Vorrichtung (1) gemäß Anspruch 4, wobei eine Mehrzahl der Spektralkomponenten eine vorgegebene Wellenlänge aufweisen, und wobei die Ringe (5) so geformt und so dimensioniert sind, dass sie einen Phasensprung bereitstellen, der gleich $2\pi p$ ist, wobei p eine ganze Zahl ist, die größer oder gleich 1 ist, und wobei p und die Breiten der Ringe (5) so ausgewählt sind, dass sie die Spektralkomponenten auf einen einzigen Brennpunkt ausrichten.

**6.** Vorrichtung (1) gemäß Anspruch 5, wobei die Ringe (5) mit Radien $r_j$ in radialem Abstand angeordnet sind, die durch Lösen der Gleichung $\Phi(r_j) = 2np$ erzielt sind, wobei $\Phi(r)$ die Phasenfunktion einer aus den optischen Ringen (5) austretenden Wellenfront repräsentiert.

**7.** Vorrichtung (1) gemäß Anspruch 1, die ausreichend Flexibilität zum anpassenden Verändern des Radius aufweist, so dass er zu der Weite der anterioren Kammer (10) passt.

**Revendications**

**1.** Dispositif de correction en seule pièce (1) pour implantation dans la chambre antérieure (10) d'un oeil phakique ou pseudophakique comprenant: une seule membrane incurvable de façon élastique ayant une épaisseur sensiblement constante d'approximativement 10 à 125 microns, et formée et dimensionnée pour s'étendre sur la chambre antérieure (10) sensiblement parallèlement à l'iris (11) ; ladite membrane comprenant une partie médiane correctrice (2), et au moins deux parties latérales (7) à cheval sur ladite partie médiane correctrice (2) ;
**caractérisé en ce que**
ladite partie médiane correctrice (2) inclut une lentille diffractive à ordre multiple, ladite lentille comprenant une zone optique discontinue (3) ayant une pluralité d'anneaux optiques concentriques (5) ; et
chacune desdites parties latérales (7) comprend au moins deux pans d'ancrage obliques (8), chacun formés et dimensionnés pour s'emboîter intimement dans un coin (9) de la chambre antérieure (10), où les pans (8) sont incurvés obliquement vers l'arrière de façon permanente de telle sorte qu'une voûte ayant une longueur sagittale d'approximativement 1 millimètre soit formée.

**2.** Dispositif (1) selon la revendication 1, dans lequel ladite membrane est faite en silicone flexible.

**3.** Dispositif (1) selon la revendication 1, dans lequel ladite lentille a une puissance de correction dans une gamme allant approximativement de moins 15 dioptries à plus 15 dioptries.

**4.** Dispositif (1) selon la revendication 1, dans lequel chacun de la pluralité d'anneaux optiques concentriques (5) a une limite d'anneau avec un anneau adjacent et lesdits anneaux (5) sont formés et dimensionnés pour fournir un saut de phase à chaque limite d'anneau pour au moins un composant spectral d'un faisceau lumineux incident sur ladite lentille.

**5.** Dispositif (1) selon la revendication 4, dans lequel une pluralité desdits composants spectraux ont une longueur d'onde donnée et lesdits anneaux (5) sont formés et dimensionnés pour fournir un saut de phase égal à $2\pi p$, dans lequel p est un entier supérieur ou égal à 1, ledit p et les largeurs des anneaux (5) sont choisis pour diriger lesdits composants spectraux vers un seul point de focalisation.

**6.** Dispositif (1) selon la revendication 5, dans lequel lesdits anneaux (5) sont espacés radialement d'un rayon, $r_j$, obtenu en résolvant l'équation $\Phi(r_j)=2\pi p$ où $\Phi(r)$ représente la fonction de phase d'un front d'onde sortant desdits anneaux optiques (5).

**7.** Dispositif (1) selon la revendication 1, ayant une flexibilité suffisante pour changer de façon ajustable ledit rayon pour correspondre à l'étendue de ladite chambre antérieure (10).

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

FIG. 15

FIG. 16

FIG. 17

FIG. 18